# EUROPÄISCHE PATENTSCHRIFT

(11) **EP 4 171 299 B1**
(45) Veröffentlichungstag und Bekanntmachung des Hinweises auf die Patenterteilung: **09.10.2024**
(21) Anmeldenummer: 21735933.0
(22) Anmeldetag: 24.06.2021
(51) Int. Cl.: A24F 40/65, H04L 67/12, A24F 40/53, A61M 15/06, G16H 40/40

(54) **TRAGBARE SYNCHRONISATIONSSTATION FÜR EINEN INHALATOR, SYNCHRONISATIONSSYSTEM UND VERFAHREN ZUR DETEKTION DES SYSTEMZUSTANDES EINES SYNCHRONISATIONSSYSTEMS**
PORTABLE SYNCHRONISATION STATION FOR AN INHALER, SYCHRONISATION SYSTEM, AND METHOD FOR DETECTING THE SYSTEM STATE OF A SYNCHRONISATION SYSTEM
STATION DE SYNCHRONISATION PORTABLE POUR UN INHALATEUR, SYSTÈME DE SYNCHRONISATION ET PROCÉDÉ DE DÉTECTION DE L'ÉTAT D'UN SYSTÈME DE SYNCHRONISATION

(30) Priorität: 29.06.2020 DE 102020117039
(43) Veröffentlichungstag der Anmeldung: 03.05.2023
(73) Patentinhaber: Körber Technologies GmbH, 21033 Hamburg (DE)
(72) Erfinder: ROSENBOHM, Arne, 21720 Steinkirchen (DE); PANKOKE, Finn-Oliver, 25436 Uetersen (DE); NIEBUHR, Gunnar, 21149 Hamburg (DE); PESAVENTO, Andreas, 58455 Witten (DE); CAPONE, Michael Joseph, 22087 Hamburg (DE)
(74) Vertreter: Müller Verweyen
(86) Internationale Anmeldenummer: PCT/EP2021/067353
(87) Internationale Veröffentlichungsnummer: WO 2022/002751

(56) Entgegenhaltungen:
- WO-A2-2020/044268
- US-A1- 2016 285 983
- US-A1- 2016 371 437

## Beschreibung

Die vorliegende Erfindung betrifft eine tragbare Synchronisationsstation für einen Inhalator, vorzugsweise für ein elektronisches Zigarettenprodukt, umfassend folgende Komponenten: einen ersten Energiespeicher, einen ersten Datenspeicher, einen ersten Prozessor, eine Aufnahme zum Einsetzen des Inhalators, und eine Inhalatorschnittstelle, die dazu eingerichtet ist, eine Datenverbindung mit dem Inhalator herzustellen, wenn der Inhalator in die Aufnahme eingesetzt ist. Des Weiteren betrifft die Erfindung ein korrespondierendes Synchronisationssystem und ein Verfahren zur Detektion des Systemzustandes eines Synchronisationssystems.

Ein großer Teil der sich auf dem Markt befindlichen Inhalatoren und elektronischen Zigarettenprodukten basiert auf dem Verdampfen von einer Flüssigkeit oder dem kontrollierten Erhitzen von Tabak. Grundsätzlich weisen Inhalatoren daher ein Heizelement zum Erhitzen des Tabaks oder der Flüssigkeit auf, das über einen Energiespeicher mit Energie versorgt wird.

Um die Handhabung von elektrischen Zigarettenprodukten für den Benutzer möglichst angenehm zu gestalten, weisen zahlreiche Zigarettenprodukte Abmessungen auf, die sich an den Abmessungen herkömmlicher Zigarettenprodukte orientieren. Ferner wird ein möglichst geringes Gewicht der elektrischen Zigarettenprodukte angestrebt, was zur Folge hat, dass die Kapazität des Energiespeichers begrenzt ist.

Es sind daher hinlänglich tragbare Basisstationen mit eigenem Energiespeicher bekannt, die mit der elektrischen Zigarette verbunden werden können. Auf diese Weise kann das elektrische Zigarettenprodukt auch unterwegs mit Energie versorgt werden.

Aus der WO 2019/108848 A1 ist eine Hülle für ein Mobiltelefon bekannt, die eine Aussparung zur Aufnahme eines elektronischen Verdampfungsgerätes aufweist. Ferner weist die Hülle einen Akku auf, über den sowohl das elektrische Verdampfungsgerät als auch das Mobiltelefon mit Energie versorgt werden kann.

Aus der US 2016/0345628 A1 ist ebenfalls eine Hülle für ein Mobiltelefon bekannt, über die eine elektrische Zigarette mit Energie versorgt werden kann. Ferner ist die Hülle dazu eingerichtet, den Akkustand oder die Anzahl der Züge an der elektrischen Zigarette an das eingesetzte Mobiltelefon zu übertragen und auf dessen Display zur Anzeige zu bringen. Somit stehen diese Informationen einem Nutzer über das Display des Mobiltelefons zur Verfügung.

Die aus diesem Stand der Technik bekannten Vorrichtungen zum Nachladen von elektrischen Zigaretten bieten allerdings keine Möglichkeit, den Systemzustand einer elektrischen Zigarette zu bestimmen.

Die WO 2020/044268 A2 offenbart eine Vorrichtung zur Diagnose des Zustandes eines elektronischen Rauchartikels. Hierzu wird eine Datenverbindung zwischen dem elektronischen Zigarettenprodukt und einem Computer hergestellt. Basierend auf den übertragenen Daten kann dann die Diagnose durchgeführt werden.

Die Aufgabe der Erfindung ist es, eine verbesserte tragbare Synchronisationsstation für einen Inhalator zum Überwachen des Systemzustandes eines Inhalators, ein entsprechend verbessertes Synchronisationssystem und ein entsprechendes Verfahren anzugeben.

Die Erfindung löst die Aufgabe mit den Merkmalen der unabhängigen Ansprüche.

Erfindungsgemäß wird vorgeschlagen, dass als weitere Komponente eine Drahtlosschnittstelle vorgesehen ist, die dazu eingerichtet ist, Daten an eine entfernte Basisstation zu übermitteln, wobei die Synchronisationsstation dazu eingerichtet ist, die Drahtlosschnittstelle derart zu steuern, dass regelmäßig automatische Verbindungsversuche mit der Basisstation unternommen werden, und eine definierte Datenmenge der in dem ersten Datenspeicher gespeicherten Daten an die Basisstation übermittelt wird, sobald eine Verbindung mit der Basisstation besteht.

Zunächst werden einige in dieser Anmeldung verwendeten Begriffe erläutert.

Unter einem automatischen Verbindungsversuch im Sinne dieser Anmeldung ist zu verstehen, dass der Verbindungsversuch ohne eine Betätigung durch einen Benutzer erfolgt.

Unter einer entfernten Basisstation ist eine Station zu verstehen, die wenigstens 5 Meter von der Synchronisationsstation entfernt ist.

Durch die von der Synchronisationsstation unternommenen automatischen Verbindungsversuche wird diese zu einer aktiven Kommunikationseinrichtung, so dass die Daten unabhängig von einer manuellen Handlung durch einen Benutzer an eine entfernte Basisstation übermittelt werden können. Auf diese Weise können in regelmäßigen Abständen Daten, beispielsweise Systemzuverlässigkeitsparameter, durch eine mit der Basisstation verbundene Auswerteeinrichtung mit entsprechender Rechenleistung ausgewertet werden, um damit zuverlässige Aussagen über den technischen Zustand des Inhalators oder der Synchronisationsstation zu treffen. Bei der Auswerteeinrichtung handelt es sich vorzugsweise um eine stationäre Einrichtung, die mit der Basisstation beispielsweise über ein Netzwerk verbunden ist.

Vorzugsweise erfolgt die Übermittlung der Daten unmittelbar von der Drahtlosschnittstelle an die Basisstation, d.h. die Daten werden ohne einen Umweg über ein Nutzerendgerät, wie beispielsweise ein Mobiltelefon, von der Drahtlosschnittstelle an die Basisstation übermittelt.

Damit der Zustand des Inhalators analysiert werden kann, müssen die Daten auch Daten des Inhalators umfassen, die zuvor von dem Inhalator auf den ersten Datenspeicher der Synchronisationsstation übertragen wurden. Die Übermittlung einer vordefinierten Datenmenge erlaubt es, bei Reduzierung des zu übermittelnden Datenvolumens dennoch zuverlässige Aussagen über den Systemzustand zu treffen. Vorzugsweise wird also nur eine Teilmenge der auf dem ersten Datenspeicher gespeicherten Daten an die Basisstation übermittelt.

Vorzugsweise ist die Synchronisationsstation dazu eingerichtet, die bereits über die Drahtlosschnittstelle an die Basisstation übermittelten Daten auf dem ersten Datenspeicher kenntlich zu machen, und bei einem erneuten Verbindungsaufbau der Drahtlosschnittstelle mit der Basisstation nur die bislang noch nicht übermittelten Daten an die Basisstation zu übermitteln. Durch eine derartige Konfiguration der Synchronisationsstation ist sichergestellt, dass Datensätze nicht mehrfach an die Basisstation übertragen werden. Das bietet den Vorteil, dass die Datenübermittlung auf ein notwendiges Maß beschränkt werden kann, so dass auch bei einer geringen Verbindungsdauer zwischen der Drahtlosschnittstelle und der Basisstation eine vollständige Übertragung der Daten möglich ist.

Erfindungsgemäß ist die Synchronisationsstation dazu eingerichtet, die auf dem ersten Datenspeicher gespeicherten Daten mit einem Zeitstempel zu versehen. Der Zeitstempel umfasst vorzugsweise neben der Uhrzeit auch das Datum, an dem die Daten von dem Inhalator an den ersten Datenspeicher übertragen werden. Mittels des Zeitstempels kann auch im Nachhinein eine zeitliche Zuordnung der Daten erfolgen. Dies kann insbesondere dann vorteilhaft sein, wenn die historische Entwicklung eines bestimmten Parameters in dem Datensatz analysiert werden soll. Weiterhin hat es sich als vorteilhaft erwiesen, die Daten erst beim Vorgang der Übertragung auf die Synchronisationsstation mit dem Zeitstempel zu versehen, weil dadurch der Inhalator einfacher und kostengünstiger ausgeführt werden kann. Dadurch, dass der Inhalator ohnehin nach dem Einsatz regelmäßig durch den Benutzer zurück in die Aufnahme der Synchronisationsstation geführt wird, ist eine Verknüpfung der Daten mit einem Zeitstempel zu diesem Zeitpunkt ausreichend.

Es wird weiter vorgeschlagen, dass die Synchronisationsstation dazu eingerichtet ist, als unabhängiges Internetendgerät betrieben zu werden. Damit kann die Synchronisationsstation unabhängig von weiteren Nutzerendgeräten, wie beispielsweise einem Mobiltelefon, eine Verbindung mit einem Internetzugangspunk, beispielsweise mit einem WLAN-Router oder einem sogenannten Wireless Access Point, herstellen. Für den Benutzer vereinfacht sich dadurch die Handhabung, weil er die Synchronisationsstation nicht mit externen Geräten koppeln muss. Ferner kann die Synchronisationsstation dadurch unabhängig vom Standort mit dem Internet verbunden werden, sofern eine Verbindung zwischen der Drahtlosschnittstelle und der Basisstation besteht und gleichzeitig die Basisstation eine Verbindung mit dem Internet ermöglicht. Es besteht somit auch die Möglichkeit, die Daten über das Internet an einen zentralen Server, der dann die Auswerteeinrichtung bildet, zu übermitteln. Über die zentrale Auswerteeinrichtung können die Daten dann analysiert werden, so dass Rückschlüsse auf den Systemzustand der Synchronisationsstation oder des Inhalators möglich sind. Ferner bietet die Ausgestaltung der Synchronisationsstation als Internetendgerät die Möglichkeit, dass Softwareaktualisierungen, beispielsweise Firmware-Updates, automatisch über die Drahtlosschnittstelle auf dem ersten Datenspeicher gesichert werden können und anschließend eine automatische Installation der neuen Software erfolgen kann. Eine Handlung des Benutzers, beispielsweise zur Durchführung der Installation, ist nicht erforderlich. Grundsätzlich ist es auch möglich, entsprechende Updates auf dem Inhalator zu installieren. Die Installationsdaten werden dann über die Inhalatorschnittstelle auf den zweiten Datenspeicher übertragen, so dass dort eine automatische Installation durchgeführt werden kann. Ferner ist vorzugsweise eine Datensicherungseinrichtung vorgesehen, um zu vermeiden, das Schadsoftware auf der Synchronisationsstation und/oder dem Inhalator installiert wird.

Gemäß einer weiteren Ausführungsform wird vorgeschlagen, dass die Daten einen oder mehrere der nachfolgenden Systemzuverlässigkeitsparameter umfassen: Betriebsdauer des Inhalators und/oder der Synchronisationsstation, Benutzungszyklen des Inhalators und/oder der Synchronisationsstation, Betriebstemperatur des Inhalators und/oder der Synchronisationsstation, Ladezyklen des Inhalators und/oder der Synchronisationsstation, Ladezustand des Inhalators und/oder der Synchronisationsstation, Betriebsdruck des Inhalators, und/oder Durchflussmenge des Inhalators. Es hat sich gezeigt, dass diese Systemzuverlässigkeitsparameter vorteilhaft Rückschlüsse auf den Systemzustand der Synchronisationsstation und/oder des Inhalators zulassen. Der Betriebsdruck des Inhalators kann dabei an verschiedenen Stellen gemessen werden. Ferner kann auch die Durchflussmenge des Inhalators, beispielsweise in Form eines Luftvolumenstroms, an verschiedenen Stellen des Inhalators gemessen werden.

Es wird weiter vorgeschlagen, dass die Drahtlosschnittstelle eine oder mehrere der folgenden Schnittstellen umfasst: eine Mobilfunkschnittstelle, eine Bluetooth-Schnittstelle und/oder eine WiFi-Schnittstelle. Vorzugsweise sind alle drei der genannten Schnittstellen vorgesehen, was verbesserte Verbindungschancen ermöglicht. So kann beispielsweise beim Fehlen eine Verbindung mit einem WLAN-Router versucht werden, eine Verbindung über die Mobilfunkschnittstelle herzustellen. Die Mobilfunkschnittstelle kann beispielsweise ein sogenanntes 5G-Modul umfassen. Selbstverständlich kann die Mobilfunkschnittstelle auch nach einem anderen gängigen Standard arbeiten. Die Bluetooth Verbindung kann beispielsweise genutzt werden, um dem Benutzer über sein Mobiltelefon elektronischen Zugriff auf die Synchronisationsstation zu ermöglichen. Dies kann beispielsweise dazu dienen, Zugangsdaten, beispielsweise die eines WLAN-Routers, einzugeben. Sofern diese Zugangsdaten einmal gespeichert sind, kann sich die Synchronisationsstation unabhängig von dem Mobiltelefon mit dem WLAN-Router verbinden.

Es wird weiter vorgeschlagen, dass die Verbindungsinformationen zum Herstellen der Verbindung zwischen der Drahtlosschnittstelle und der Basisstation vorgespeichert sind. Unter einer vorgespeicherten Information im Sinne dieser Anmeldung ist zu verstehen, dass diese bereits bei der Auslieferung der Synchronisationsstation, beispielsweise auf dem ersten Datenspeicher, gespeichert sind. Vorzugsweise sind dann allerdings nicht alle Zugangsdaten für sämtliche Schnittstellen vorgespeichert, sondern nur ausgewählte Zugangsdaten. So können beispielsweise die Zugangsdaten für das Mobilfunkmodul auf einer SIM-Karte oder eSIM vorgespeichert sein, oder die Zugangsdaten für bestimmte WLAN-Router beispielsweise auf dem ersten Datenspeicher.

Vorzugsweise ist eine mechanische Schaltvorrichtung vorgesehen, über die die Drahtlosschnittstelle deaktivierbar und/oder deren Verbindung zu den übrigen Komponenten trennbar ist. Somit kann der Benutzer entscheiden, ob er die Möglichkeit schaffen möchte, die Daten an die Basisstation zu übermitteln. Sofern die Drahtlosschnittstelle mehrere Drahtlosschnittstellen umfasst, ist die Schaltvorrichtung vorzugsweise dazu eingerichtet, sämtliche Schnittstellen zu deaktivieren bzw. zu trennen.

Gemäß einer weiteren Ausführungsform sind die Komponenten, also der erste Energiespeicher, der erste Datenspeicher, der erste Prozessor, die Drahtlosschnittstelle und die Aufnahme, innerhalb eines gemeinsamen Gehäuses angeordnet und mit einer gemeinsamen Trägerstruktur fest verbunden. Unter einer festen Verbindung im Sinne dieser Anmeldung ist eine Verbindung zu verstehen, die nur unter der Zuhilfenahme von Werkzeug lösbar ist. Durch die feste Anordnung der Komponenten an einer Trägerstruktur und innerhalb eines Gehäuses kann eine integrale Bauweise geschaffen werden. Diese ermöglicht erstens eine kompakte Bauweise, die die Transportfähigkeit, insbesondere in Hosen- oder Handtaschen, verbessert. Zweitens ist ein solcher Aufbau für die Zuverlässigkeit der Synchronisationsstation von Vorteil, weil die Komponenten durch das Gehäuse vor äußeren Umgebungseinflüssen geschützt sind. Drittens kann durch die Trägerstruktur eine sichere Anordnung bzw. Positionierung der Komponenten auch bei Erschütterungen oder sonstigen Umgebungseinflüssen, die beim Mitführen durch den Benutzer auftreten können, gewährleistet werden.

Das Gehäuse ist vorzugsweise dazu eingerichtet, den Inhalator im eingesetzten Zustand vollständig zu umgeben, sodass der Inhalator auf diese Weise beim Mitführen durch den Benutzer vor Umgebungseinflüssen vollständig geschützt ist und die Datenübertragung von dem Inhalator auf den ersten Datenspeicher zuverlässig erfolgen kann. Der Inhalator, insbesondere ein Mundstück des Inhalators, kann so hygienisch gelagert und vor einer unerwünschten Kontamination geschützt werden. Um die Lagerung des Inhalators in der Synchronisationsstation weiter zu verbessern, ist der Inhalator vorzugsweise verdrehsicher in der Aufnahme gelagert.

Gemäß einer weiteren Ausführungsform handelt es sich bei der Inhalatorschnittstelle um eine Kontakt-Schnittstelle, vorzugsweise um eine USB-Schnittstelle; weiter vorzugsweise handelt es sich um einen männlichen USB-Stecker im USB-C Format. Durch die USB-Schnittstelle kann eine einfache und kostengünstige Datenverbindung zwischen dem Inhalator und der Synchronisationsstation geschaffen werden, sodass insbesondere der Inhalator einen vereinfachten Aufbau aufweisen kann. Kosten- und energieintensivere Drahtlosschnittstellen, wie beispielsweise Bluetooth-, Mobilfunk- oder WiFi-Module, können damit zur Verbindung des Inhalators mit der Synchronisationsstation entfallen. Ferner ist es vorteilhaft, dass über die USB-Schnittstelle der Inhalator auch mit Energie versorgt werden kann.

Die eingangs genannte Aufgabe wird ebenfalls gelöst durch ein tragbares Synchronisationssystem umfassend die Synchronisationsstation nach einem der Ansprüche 1 bis 10 und einen Inhalator, umfassend einen zweiten Energiespeicher, einen zweiten Prozessor und einen zweiten Datenspeicher. Das Synchronisationssystem ermöglicht es, Daten des Inhalators mittels der Synchronisationsstation an die Basisstation zu übermitteln. Der Inhalator selbst muss nicht mit einer Drahtlosschnittstelle ausgestattet werden, so dass dieser einen kompakten und kostengünstigen Aufbau aufweisen kann. Zwischen dem Inhalator und der Synchronisationsstation wird lediglich eine einzige Schnittstelle benötigt. Das energieaufwendige Senden und Empfangen von Daten über die Drahtlosschnittstelle an die entfernte Basisstation kann von der Synchronisationsstation übernommen werden.

Vorzugsweise entspricht das Speichervolumen des ersten Datenspeichers mindestens dem 10-fachen des Speichervolumens des zweiten Datenspeichers, weiter vorzugsweise mindestens dem 80-fachen, insbesondere vorzugsweise mindestens dem 100-fachen. Es hat sich gezeigt, dass diese Speichergröße vorteilhaft ist, um die Daten des Inhalators ausreichend lange zwischenzuspeichern, bis eine Übertragung an die Basisstation möglich ist. Das Speichervolumen kann vorteilhafterweise so groß gewählt werden, dass sämtliche Daten für die typische Lebensdauer eines Inhalators auf dem ersten Datenspeicher gesichert werden können. Dies ermöglicht auch dann eine vollständige Sicherung der Daten, wenn sich der Benutzer über einen längeren Zeitraum hinweg in einer Region befindet, in der ein Verbindungsaufbau der Drahtlosschnittstelle mit einer Basisstation nicht möglich ist. Bei dem ersten Datenspeicher handelt es sich beispielsweise um einen Solid-State-Drive (SSD). Dementsprechend muss der zweite Datenspeicher des Inhalators nur ein Speichervolumen aufweisen, das ausreicht, um die Daten von vorzugsweise maximal 30, weiter vorzugsweise maximal 20 Inhalationszügen zu speichern; dies entspricht der maximalen Anzahl der Züge pro Inhalationseinheit. Dadurch können die Kosten und die Größe des Inhalators reduziert werden.

In entsprechender Weise ist es vorteilhaft, wenn die Kapazität des ersten Energiespeichers mindestens dem 10-fachen der Kapazität des zweiten Energiespeichers entspricht, weiter vorzugsweise mindestens dem 80-fachen und insbesondere vorzugsweise dem 100-fachen. Es hat sich gezeigt, dass dadurch ein vorteilhafter Kompromiss zwischen Gewicht und Abmessungen der Synchronisationsstation sowie eine ausreichende Energieversorgung für die entsprechende Anzahl an Ladezyklen des Inhalators erreicht werden kann.

Vorzugsweise ist das Synchronisationssystem dazu eingerichtet, die auf dem zweiten Datenspeicher gespeicherten Daten über die Inhalatorschnittstelle automatisch auf den ersten Datenspeicher zu übertragen, wenn der Inhalator in die Aufnahme eingesetzt ist. Dadurch, dass die Daten bei jedem Einsetzen an die Synchronisationsstation übertragen werden, kann der zweite Datenspeicher des Inhalators ein geringes Speichervolumen aufweisen, sodass ein kompakter und kostengünstiger Aufbau des Inhalators möglich ist. Vorzugsweise werden die Daten von dem zweiten Datenspeicher auf den ersten Datenspeicher im eingesetzten Zustand vollständig übertragen und weiter vorzugsweise nach der erfolgreichen Datenübertragung von dem zweiten Datenspeicher gelöscht. Ferner ergibt sich daraus der Vorteil, dass in geringen Zeitabständen die auf dem ersten Datenspeicher gesicherten Daten während des Übertragungsvorgangs auf den ersten Datenspeicher mit einem Zeitstempel versehen werden können, sodass der durch die Synchronisationsstation vergebene Zeitstempel möglichst nahe an dem Zeitpunkt liegt, an dem die Daten tatsächlich von dem Inhalator erfasst worden sind.

Vorzugsweise wird der Inhalator über die Inhalatorschnittstelle mit Energie versorgt, wenn der Inhalator in die Aufnahme eingesetzt ist. Auf diese Weise kann sichergestellt werden, dass der zweite Energiespeicher des Inhalators auch bei häufigem Gebrauch ausreichend geladen ist.

Die eingangs genannte Aufgabe wird ferner gelöst durch ein Verfahren zur Detektion des Systemzustands eines Synchronisationssystems nach einem der Ansprüche 11 bis 13, wobei in einem ersten Verfahrensschritt wenigstens ein Systemzuverlässigkeitsparameter des Inhalators automatisch über die Inhalatorschnittstelle an die Synchronisationsstation übermittelt und auf dem ersten Datenspeicher gespeichert wird, wenn der Inhalator in die Aufnahme eingesetzt ist; in einem zweiten Verfahrensschritt die Systemzuverlässigkeitsparameter des Inhalators mit einem Zeitstempel versehen werden und diese in einem zu versendenden Datenpaket zusammengefasst werden; in einem dritten Verfahrensschritt das Datenpaket über die Drahtlosschnittstelle und die Basisstation an eine Auswerteeinrichtung übermittelt wird; und in einem vierten Verfahrensschritt eine Auswertung des Datenpakets erfolgt und in Abhängigkeit eines so detektierten Systemzustandes eine Handlungsaufforderung generiert wird.

Das Verfahren ermöglicht es, die Daten in einer Auswerteeinrichtung zu verarbeiten, die vorzugsweise eine signifikant höhere Rechenleistung als der erste Prozessor der Synchronisationsstation aufweist. Somit können auch komplexere Auswertungen der Daten vorgenommen werden, die eine hohe Rechenleistung erfordern. Insbesondere kann ein Abgleich mit historischen Daten innerhalb einer Datenbank vorgenommen, der zeitliche Verlauf der Daten analysiert und/oder ein Vergleich mit Soll- bzw. Schwellwerten vorgenommen werden. Auf Basis der Auswertung wird eine Handlungsempfehlung vorgeschlagen, durch deren Umsetzung insgesamt der Systemzustand des Synchronisationssystems, insbesondere des Inhalators, verbessert werden kann. Vorzugsweise können die an die Auswerteeinrichtung übermittelten Daten und/oder die Handlungsempfehlungen über ein Onlineportal durch den Benutzer eingesehen werden.

Die Auswerteeinrichtung kann beispielsweise mehr als 1 km entfernt von der Synchronisationsstation angeordnet sein, beispielsweise 10 km entfernt. In anderen Ausführungsformen kann die Entfernung beispielsweise auch mehr als 100 km betragen.

Es wird weiter vorgeschlagen, dass in einem fünften Verfahrensschritt die Handlungsaufforderung an die Synchronisationsstation übermittelt wird. Die Handlungsempfehlung kann dort einem Benutzer über eine Ausgabeeinrichtung zur Anzeige gebracht werden. Die Ausgabeeinrichtung kann beispielsweise durch eine LED gebildet sein, wobei jeweils eine Leuchtfarbe der LED für den Benutzer eine Handlungsaufforderung kennzeichnet. Beispielsweise kann in einem kritischen Systemzustand die Handlungsempfehlungen erfolgen, die Synchronisationsstation und/oder den Inhalator nicht mehr zu benutzen. Ferner kann beispielsweise eine Handlungsempfehlungen lauten, dass der Inhalator gereinigt werden muss oder eine Instandhaltung durchzuführen ist. Durch diese Handlungsempfehlungen kann zum einen die Sicherheit für den Benutzer erhöht werden und zum anderen eine gleichbleibende Qualität bei der Benutzung des Inhalators während der gesamten Lebensdauer des Inhalators sichergestellt werden. In einem letzten Verfahrensschritt erfolgt dann eine Umsetzung der Handlungsempfehlung, so dass der Systemzustand des Synchronisationssystems verbessert wird.

Alternativ oder zusätzlich kann in einem letzten Verfahrensschritt die Handlungsempfehlung auch automatisch umgesetzt werden. So kann ein kritischer Systemzustand eine Deaktivierung der Synchronisationsstation und/oder des Inhalators zur Folge haben. Ferner kann es auch zur Anpassung von bestimmten Verdampfungsparametern kommen. Es könnte beispielsweise die Heizleistung eines Heizers des Inhalators angepasst werden oder eine Selbstreinigung des Inhalators durchgeführt werden.

Die Erfindung wird im Folgenden anhand bevorzugter Ausführungsformen unter Bezugnahme auf die beigefügten Figuren erläutert. Dabei zeigt
- Fig. 1: eine schematische Darstellung einer Synchronisations-station mit einem geöffneten Gehäuse;
- Fig. 2: eine perspektivische Ansicht eines Inhalators;
- Fig. 3: eine schematische Darstellung einer Synchronisations-station, eine Basisstation und eine Auswerteeinrich-tung;
- Fig. 4: eine perspektivische Ansicht eines tragbaren Synchro-nisationssystems in einer ersten Ausführungsform;
- Fig. 5: eine perspektivische Ansicht eines tragbaren Synchro-nisationssystems in einer zweiten Ausführungsform mit einem nicht eingesetzten Inhalator;
- Fig. 6: eine perspektivische Ansicht eines tragbaren Synchronisationssystems in einer zweiten Ausführungsform mit einem eingesetzten Inhalator; und
- Fig. 7: ein Verfahren zur Detektion des Systemzustands eines Synchronisationssystems.

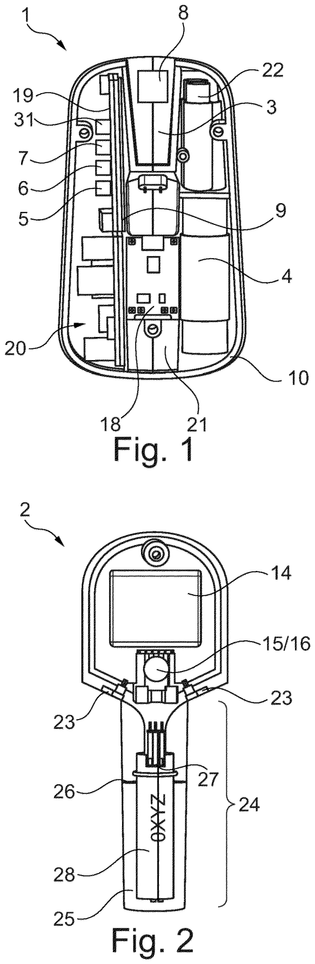

Figur 1 zeigt schematisch eine geöffnete Synchronisationsstation 1, bei der zur Veranschaulichung des Aufbaus eine Vorderseite eines Gehäuses 10 entfernt wurde. Die Synchronisationsstation 1 umfasst als Komponenten eine Aufnahme 3 zum Einsetzen eines Inhalators 2 (siehe Figur 2), einen ersten Energiespeicher 4, einen ersten Datenspeicher 5, einen ersten Prozessor 6, eine Inhalatorschnittstelle 8 und eine Drahtlosschnittstelle 7.

Die Komponenten sind mit einer Trägerstruktur 9 fest verbunden, also nur mittels Werkzeug lösbar, und innerhalb des Gehäuses 10 angeordnet, so dass die Synchronisationsstation 1 einen kompakten Aufbau aufweist. Das Gehäuse 10 ist beispielsweise aus Kunststoff gefertigt. Die Trägerstruktur 9 ist vorzugsweise durch ein separates Bauteil gebildet, kann aber auch ganz oder teilweise durch einen Teil des Gehäuses 10 gebildet sein.

Die Aufnahme 3 ist derart ausgestaltet, dass sie den Inhalator 2, beispielsweise in Form einer elektrischen Zigarette oder eines medizinischen Inhalators 2, zumindest teilweise darin aufnehmen kann. In dem Ausführungsbeispiel gemäß Figur 1 ist die Aufnahme 3 derart ausgeführt, dass der Inhalator 2 im eingesetzten Zustand zu mehr als 1/3 seiner länglichen Erstreckung in die Aufnahme 3 hineinragt. Um den Inhalator 2 im eingesetzten Zustand zuverlässig lagern zu können, sind beispielsweise Sicherungselemente vorgesehen. Das Sicherungselement kann beispielsweise durch einen Magneten gebildet sein, der den Inhalator 2 in der Aufnahme 3 hält, und/oder durch ein Sicherungselement, das den Inhalator 2 in der Aufnahme 3 verdrehsicher lagert.

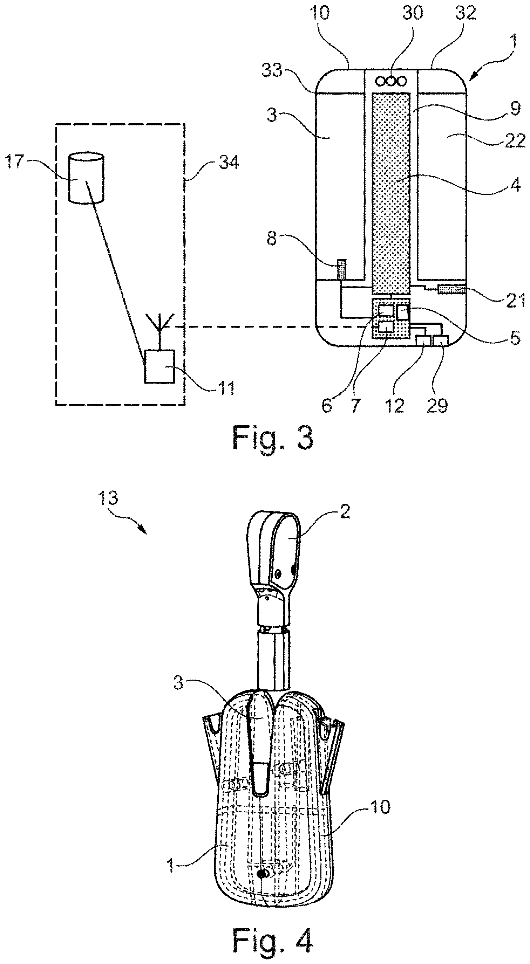

Im eingesetzten Zustand ist der Inhalator 2 mit dem ersten Datenspeicher 5 und mit einem Batteriemanagementsystem 18 verbunden. Die Synchronisationsstation 1 ist dazu eingerichtet die auf dem Inhalator 2 gespeicherten Daten vollständig auf den ersten Datenspeicher 5 zu übertragen, sobald der Inhalator 2 in der Aufnahme 3 positioniert ist. Dieser Vorgang kann beispielsweise mittels des ersten Prozessors 6 gesteuert werden. Über die Drahtlosschnittstelle 7 können die auf dem ersten Datenspeicher 5 gespeicherten Daten dann an eine entfernte Basisstation 11 (siehe Figur 3) übermittelt werden.

Die Drahtlosschnittstelle 7 umfasst ein WiFi-Modul, ein Mobilfunkmodul und ein Bluetooth-Modul. Die Drahtlosschnittstelle 7 wird vorzugsweise von dem ersten Prozessor 6 derart gesteuert, dass regelmäßig automatische Verbindungsversuche zu der Basisstation 11 unternommen werden. Dies kann beispielsweise in bestimmten Zeitintervallen, beispielsweise einmal pro Stunde, der Fall sein, oder Ereignisgesteuert, beispielsweise nach einer Übertragung der Daten auf den ersten Datenspeicher. Ferner werden die auf dem ersten Datenspeicher 5 gesendeten Daten automatisch an die Basisstation 11 übermittelt, sobald eine Verbindung zwischen der Drahtlosschnittstelle 7 und der Basisstation 11 aufgebaut ist.

Der erste Datenspeicher 5, der erste Prozessor 6 und die Drahtlosschnittstelle 7 sind auf einer gemeinsamen Leiterplatte 19 angeordnet; sie bilden zusammen ein Datenmanagementsystem. Sowohl die automatische Speicherung der Daten des Inhalators 2 auf dem ersten Datenspeicher 5, der automatisierte Verbindungsaufbau mit der Basisstation 11 als auch die automatische Übermittlung der Daten an die Basisstation 11 erfolgt vorzugsweise durch einen auf dem ersten Datenspeicher 5 gespeicherten Programmcode, der durch den ersten Prozessor 6 ausführbar ist. Ferner ist auf der Leiterplatte 19 auch ein Element zur Ansteuerung der Ausgabeeinrichtung 30 (siehe Figur 3), die beispielsweise durch LEDs und/oder ein Display gebildet ist, vorgesehen.

Die Inhalatorschnittstelle 8 ist vorzugsweise durch eine männliche USB-Schnittstelle im USB-C Format gebildet, sodass eine kostengünstige und einfache Datenverbindung zu dem Datenmanagementsystem und auch zu dem ersten Energiespeicher 4 möglich ist. Alternativ kann die Datenverbindung zwischen der Synchronisationsstation 1 und dem Inhalator 2 auch über eine optische Signalübertragungseinrichtung der über NFC erfolgen.

Auf der Leiterplatte 19 kann ferner auch eine Hardware-Uhr 31 vorgesehen sein, deren Uhrzeit und Datumsinformation genutzt werden kann, um Datensätze mit einem Zeitstempel zu versehen.

Über den ersten Energiespeicher 4 kann ein zweiter Energiespeicher 14 des Inhalators 2 mit Energie versorgt werden. Das Batteriemanagementsystem 18 ist sowohl dazu eingerichtet ist, den ersten Energiespeicher 4 der Synchronisationsstation 1 als auch den zweiten Speicher 14 des Inhalators 2 zu steuern, zu regeln bzw. zu überwachen.

Bei dem ersten und zweiten Energiespeicher 4 und 14 handelt es sich bei diesem Ausführungsbeispiel um einen wiederaufladbaren Akku. Ferner ist ein Aufwärtswandler 20 vorgesehen, über den die Ladespannung für den zweiten Energiespeicher 14 bereitgestellt werden kann. So kann eine Spannung des ersten Energiespeichers 4, die beispielsweise zwischen 3,7 - 4,2 V beträgt, auf eine Ladespannung von mehr als 5 V erhöht werden.

Um den ersten Energiespeicher 4 mit Energie zu versorgen, ist ferner eine Ladeschnittstelle 21 vorgesehen, die vorzugsweise durch eine weibliche USB-Schnittstelle, weiter vorzugsweise durch eine USB-C-Schnittstelle, gebildet ist. Alternativ oder zusätzlich kann auch ein Solarzellenmodul vorgesehen sein, um den ersten Energiespeicher 4 mit Energie zu versorgen.

Die Synchronisationsstation 1 umfasst eine Aufbewahrungseinrichtung 22 die dazu eingerichtet ist, Verbrauchsmaterialien für den Inhalator 2, also beispielsweise sogenannte Pods oder Tabaksticks, aufzubewahren. Ferner kann die Aufbewahrungsrichtung 22 beispielsweise dazu genutzt werden, ein Ladekabel darin zu transportieren.

Figur 2 zeigt einen Inhalator 2 der dazu eingerichtet ist, mit einem Teilabschnitt 24 in die Aufnahme 3 der Synchronisationsstation 1 aus Figur 1 eingesetzt zu werden. Der Inhalator 2 umfasst ein Mundstück 25, das über ein lösbares Verbindungsmittel 26, beispielsweise über einen Bajonettverschluss, mit dem übrigen Teil des Inhalators 2 verbunden ist. In dem Mundstück 25 ist eine Kartusche 28 gelagert, in der sich ein zu verdampfendes oder zu erhitzendes Medium befindet, das über ein Heizelement 27 erhitzbar ist. Das lösbare Verbindungsmittel 26 erlaubt es, die Kartusche 28 einfach und komfortabel zu wechseln. Bei dem Medium handelt es sich beispielsweise um einen Tabak oder ein Liquid.

Der Inhalator 2 kann ferner auch eine nicht dargestellte Leseeinrichtung aufweisen, über die eine ID oder Seriennummer der eingesetzten Kartusche 28 ausgelesen werden kann. Über diese Information kann beispielsweise das zu erhitzende Medium durch den Inhalator 2 identifiziert werden, so dass die Erhitzungsparameter des Heizelements 27 entsprechend eingestellt werden können. Ferner kann beispielsweise durch ein defektes elektronisches Siegel der Kartusche 28 eine unbrauchbare Kartusche detektiert werden. Die Verwendung der defekten Kartusche 28 kann dann unterbunden werden oder der Benutzer durch die Ausgabeeinrichtung 30 darauf hingewiesen werden.

Zur Energieversorgung des Inhalators 2, insbesondere eines zweiten Prozessors 16 und des Heizelements 27, ist ein zweiter Energiespeicher 14 vorgesehen. Der zweite Energiespeicher 14 hat eine Kapazität, die ausreicht, um Energie für 15 bis 25 Inhalationszüge bereitzustellen, vorzugsweise für 20 Inhalationszüge. Durch diese Auslegung des zweiten Energiespeichers 14 kann eine kleine und leichte Bauform des Inhalators 2 ermöglicht werden.

Der Inhalator 2 umfasst ferner einen zweiten Datenspeicher 15 der gemeinsam mit dem zweiten Prozessor 16 auf einer Leiterplatte angeordnet ist. Beim Inhalationsvorgang generierte Daten werden auf dem zweiten Datenspeicher 15 gespeichert.

Ferner sind der zweite Datenspeicher 15 und der zweite Prozessor 16 dazu eingerichtet, mit einer Ausgabeeinrichtung des Inhalators 2, beispielsweise in Form von LEDs, mit einer Bluetooth-Schnittstelle, und/oder mit Sensoren, beispielsweise Druck- bzw. Durchflusssensoren zu kommunizieren.

Mittels der Bluetooth-Schnittstelle kann der Inhalator 2 direkt mit einem Smartphone verbunden werden, sodass darüber beispielsweise die Intensität, also der Grad der Beimischung des zu erhitzenden oder zu verdampfenden Mediums, beim Inhalieren eingestellt werden kann.

Die Kommunikation mit den Sensoren erfolgt derart, dass diese während des Inhalationsvorgangs aktiviert sind, sodass diese Sensordaten generieren können, die dann auf dem zweiten Datenspeicher 16 gespeichert werden.

Zusätzlich kann der Inhalator 2 einen Beschleunigungssensor umfassen, dessen Daten ebenfalls auf dem zweiten Datenspeicher 15 gesichert werden können. Mittels des Beschleunigungssensors lassen sich beispielsweise Gesten erkennen.

Ferner kann der Inhalator 2 dazu eingerichtet sein, ein Herunterfallen des Inhalators 2 zu detektieren. Zur Detektion eines Herunterfallens bzw. des daraus resultierenden Aufprallstoßes können beispielsweise die Daten des Beschleunigungssensor genutzt werden. Diese Daten können dann beispielsweise mittels des zweiten Prozessors 16 ausgewertet werden, so dass ein Herunterfallen vom Inhalator 2 selbst detektiert werden kann. Alternativ kann der Inhalator 2 auch eine separate Detektionseinrichtung zur Detektion eines Herunterfallens umfassen. Durch die Detektion eines Herunterfallens oder eines Stoßes können Ursachen von Systemveränderungen zuverlässiger ermittelt werden. Vorzugsweise werden auch die Informationen über das Herunterfallen oder über die Stöße von dem Inhalator 2 auf den ersten Datenspeicher 5 übertragen, sobald der Inhalator 2 in der Aufnahme 3 positioniert ist.

Ferner kann in dem Inhalator 2 ein Vibrationsmodul vorgesehen sein, über das der gesamte Inhalator 2 in Vibration versetzt werden kann, sodass darüber ebenfalls eine Kommunikation mit dem Nutzer möglich ist. So kann beispielsweise eine maximale Anzahl an Inhalationszügen pro Zeiteinheit eingestellt werden und bei Überschreiten dieser Anzahl der Benutzer durch eine entsprechende Vibration des Inhalators 2 darauf hingewiesen werden.

Um die Daten von dem zweiten Datenspeicher 15 auf den ersten Datenspeicher 5 der Synchronisationsstation 1 zu übertragen, ist eine Schnittstelle 23 an dem Inhalator 2 vorgesehen. Es handelt sich dabei um eine weibliche USB-C-Schnittstelle, so dass diese mit der Inhalatorschnittstelle 8 der Synchronisationsstation 1 zusammenwirken kann. Vorzugsweise weist der Inhalator 2 zwei Schnittstellen 23 auf, die in Bezug auf eine Längsachse des Inhalators 2 um 180° versetzt angeordnet sind; so kann auch bei einem verdrehten Einsetzen des Inhalators 2 in die Aufnahme 3 eine Verbindung der Schnittstelle 23 mit der Inhalatorschnittstelle 8 hergestellt werden. Ferner kann über die Schnittstelle 23 auch der zweite Energiespeicher 14 mit Energie versorgt werden.

Figur 3 zeigt eine zweite Ausführungsform der Synchronisationsstation 1 mit einem im Vergleich zu der Ausführungsform gemäß Figur 1 abweichenden Aufbau. Die bereits von der Synchronisationsstation 1 aus Figur 1 bekannten Funktionalitäten bleiben jedoch erhalten. Es wird daher nachfolgend nur auf die Unterschiede im Vergleich zu der Synchronisationsstation 1 aus der Figur 1 eingegangen.

Für den Inhalator 2 ist die Aufnahme 3 an einem linken Rand vorgesehen. Die Aufnahme 3 ist durch einen Deckel 32 vollständig verschließbar, sodass der Inhalator 2 hygienisch darin transportiert werden kann. Der Deckel 32 ist an dem Gehäuse 10 über einen Scharnier 33 gelagert, sodass die Aufnahme 3 durch eine Klappbewegung des Deckels 32 geöffnet und geschlossen werden kann. In gleicher Weise ist auch die Aufbewahrungseinrichtung 22 durch einen Deckel 32 verschließbar. Der Deckel 32 kann durch eine nicht dargestellte Sicherungseinrichtung vor einem unerwünschten Öffnen gesichert werden.

Figur 3 zeigt auch die Inhalatorschnittstelle 8, die dazu eingerichtet ist, den Inhalator 2 daten- und energietechnisch mit der Synchronisationsstation 1 zu verbinden. Der erste Energiespeicher 4 ist bei dieser Ausführungsform zwischen der Aufnahme 3 und der Aufbewahrungsrichtung 22 angeordnet. Der erste Energiespeicher ist über eine externe Ladeschnittstelle 21, die über das Gehäuse 10 zugänglich ist, mit Energie versorgbar. Die externe Ladeschnittstelle 21 ist als weiblicher USB-C Anschluss ausgestaltet.

Ferner ist in Figur 3 die Drahtlosschnittstelle 7 in einem Zustand dargestellt, in dem deren Mobilfunkschnittstelle mit einer entfernten Basisstation 11 verbunden ist. Die Basisstation 11 ist in diesem Fall eine Mobilfunkstation, die beispielsweise zur Kommunikation über einen der Dienste GSM, GPRS, 3G, HSDPA, UMTS, LTE, 5G und/oder darauf basierenden Diensten eingerichtet ist. Die Kommunikation zwischen der Drahtlosschnittstelle 7 und der Basisstation 11 kann über einen der folgenden Dienste, Standards und/oder Protokolle erfolgen: LPWAN SIGFOX, clean state, Narrowband loT, NB LTW-M, LTE-M, EC-GSM, 5G. Die Identifikation kann beispielsweise durch eine SIM-Karte oder durch eine eSIM in der Synchronisationsstation 1 erfolgen, so dass sich die Synchronisationsstation 1 beispielsweise in ein serverseitige Netzwerk 34, das auch die Basisstation 11 umfasst, einwählen kann.

Über die drahtlose Verbindung zwischen der Drahtlosschnittstelle 7 und der Basisstation 11 sind Daten des ersten Datenspeichers 5 an die Basisstation 11 übermittelbar. Die Basisstation 11 ist innerhalb des serverseitigen Netzwerks 34 mit einer Auswerteeinrichtung 17 verbunden. Die Auswerteeinrichtung 17 ist dazu eingerichtet, die übermittelten Daten, bei denen es sich beispielsweise um Systemzuverlässigkeitsparameter handeln kann, zu analysieren, so dass Rückschlüsse auf den Systemzustand des Inhalators 2 möglich sind.

Die Drahtlosschnittstelle 7 ist auch dazu eingerichtet, Daten von der Basisstation 11 zu empfangen. So kann beispielsweise ein durch die Auswerteeinrichtung 17 festgestellter kritischer Systemzustand in Form eines Fehlercodes über die Basisstation 11 an die Drahtlosschnittstelle 7 übermittelt werden. Die Synchronisationsstation 1 ist dazu eingerichtet, den Systemzustand über die Ausgabeeinrichtung 30 für einen Benutzer zur Anzeige zu bringen. Dies kann beispielsweise in Form einer rot leuchtenden LED erfolgen. Durch die Ausgabeeinrichtung 30 kann beispielsweise einer oder mehrere der folgenden Zustände zur Anzeige gebracht werden: Der Ladezustand des ersten Energiespeichers 4, der Ladezustand des zweiten Energiespeichers 14, der Synchronisationsstatus zwischen dem Inhalator 2 und der Synchronisationsstation 1 bzw. zwischen dem Netzwerk 34 und der Synchronisationsstation 1, der Verbindungsstatus der Synchronisationsstation 1 mit dem Netzwerk 34 und der Zustand bzw. Füllstand des zu erhitzenden oder zu verdampfenden Mediums.

Das Empfangen von Daten durch die Synchronisationsstation 1 ist auch für dessen Verwendung bei medizinischen Inhalatoren 2 vorteilhaft. Durch die Vernetzung der Synchronisationsstation 1 mit der Auswerteeinrichtung 17 ist eine präzise Steuerung der Verabreichung von Wirkstoffen und/oder der Abgabe von Dampf und/oder Aerosol und/oder eine Erinnerungsfunktion möglich. Ein Erinnerungssignal kann von der Auswerteeinrichtung 17 über die Basisstation 11 an die Synchronisationsstation 1 übermittelt werden. Das Signal wird dann beispielsweise über die Ausgabeeinrichtung 30 der Synchronisationsstation 1 für einen Benutzer zur Anzeige gebracht. Alternativ oder zusätzlich kann ein solches Erinnerungssignal auch über die Inhalatorschnittstelle 8 an den Inhalator 2 übertragen werden und dort beispielsweise über eine LED und/oder eine Vibrationseinrichtung dem Benutzer zur Anzeige gebracht werden.

Insbesondere wenn die Synchronisationsstation 1 als unabhängiges Internetendgerät ausgeführt ist, können Informationen zu einem Nutzungsregime, beispielsweise mit den Merkmalen Nutzungsdauer, Nutzungshäufigkeit und/oder die Nutzungsintensität des Inhalators 2, komfortabel an die Synchronisationsstation 1 übermittelt werden. Vor dem Start eines neuen Regimes kann die Synchronisationsstation 1 den Zustand des ersten Energiespeichers 4, des zweiten Energiespeicher 14 und/oder des zu erhitzenden Mediums überprüfen, so dass eine störungsfreie Verwendung des Inhalators 2 durch den Benutzer sichergestellt werden kann.

Die Synchronisationsstation 1 weist eine mechanische Schaltvorrichtung 12 auf, über die die Drahtlosschnittstelle 7 deaktiviert werden kann. Ferner ist ein Rücksetzschalter 29 vorgesehen, mit dem der Auslieferungszustand der Synchronisationsstation 1 wiederhergestellt werden kann.

Figur 4 zeigt eine perspektivische Ansicht eines Synchronisationssystems 13 umfassend eine Synchronisationsstation 1, die eine mittige Aufnahme 3 für den Inhalator 2 aufweist. Links und rechts von der Aufnahme 3 ist jeweils eine Aufbewahrungseinrichtung 22 vorgesehen, die durch eine Schwenkbewegung gegenüber dem Gehäuse 10 geöffnet und geschlossen werden kann.

Das Synchronisationssystem 13 kann auch dazu eingerichtet sein, in Abhängigkeit einer Seriennummer oder ID der Kartusche 28 ein Nutzungsregime zu laden. Der Benutzer kann bei der Durchführung des Regimes unterstützt werden, indem entsprechende Informationen über die Ausgabeeinrichtung 30 in Form einer Vibrationseinrichtung, einer LED, eines Displays, und/oder einer Sprachausgabeeinrichtung übermittelt werden. Die Informationen können alternativ oder zusätzlich auch durch eine Ausgabeeinrichtung des Inhalators 2 an den Benutzer übermittelt werden.

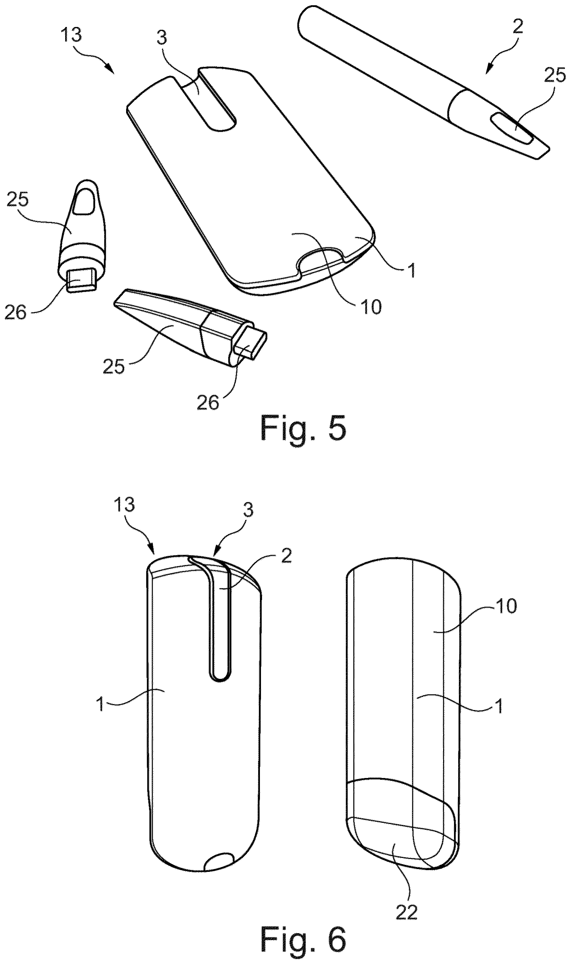

Figur 5 zeigt das Synchronisationssystem 13 gemäß einem zweiten Ausführungsbeispiel. Das Mundstück 25 des Inhalators 2 ist über ein Verbindungsmittel 26 in Form einer Steckverbindung 35 mit dem übrigen Inhalator 2 verbindbar.

Figur 6 zeigt das Synchronisationssystem 13 in der Ausführungsform aus Figur 5 in einem Zustand, in dem der Inhalator 2 in die Synchronisationsstation 1 eingesetzt ist.

Der Inhalator 2 kann von oben mittig in die Synchronisationsstation 1 eingeschoben werden, sodass der Inhalator 2 bündig mit dem Gehäuse 10 der Synchronisationsstation 1 abschließt. Der Inhalator 2 bildet bei dieser Ausführungsform einen Teil des Gehäuses 10, wenn dieser in die Aufnahme 3 eingesetzt ist. Durch eine solche Ausgestaltung kann eine besonders kompaktes und einfach transportables Synchronisationssystem 13 geschaffen werden. Bei dieser Ausführungsform ist die Aufbewahrungseinrichtung 22 rückseitig angeordnet, also an der der Aufnahme 3 für den Inhalator 2 gegenüberliegenden Seite.

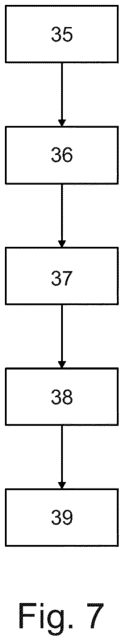

Figur 7 zeigt schematisch den Ablauf eines Verfahrens zur Detektion des Systemzustandes des Synchronisationssystems 13.

In einem ersten Verfahrensschritt 35 wird wenigstens ein Systemzuverlässigkeitsparameter des Inhalators 2 automatisch über die Inhalatorschnittstelle 8 an die Synchronisationsstation 1 übermittelt und auf dem ersten Datenspeicher 5 gespeichert, wenn der Inhalator 2 in die Aufnahme 3 eingesetzt ist. Nach dem Übermitteln der Systemzuverlässigkeitsparameter an den ersten Datenspeicher 5 werden die Daten auf dem zweiten Datenspeicher 15 des Inhalators 2 dann zumindest teilweise gelöscht.

In einem zweiten Verfahrensschritt 36 werden die Systemzuverlässigkeitsparameter des Inhalators 2 mit einem Zeitstempel versehen und diese in einem zu versendenden Datenpaket zusammengefasst. Dieses Datenpaket wird zunächst in dem ersten Datenspeicher 5 gespeichert, bis durch die Drahtlosschnittstelle 7 eine Verbindung mit der Basisstation 11 besteht.

Erst dann erfolgt in einem dritten Verfahrensschritt 37 eine Übermittlung des Datenpakets über die Drahtlosschnittstelle 7 und die Basisstation 11 an die Auswerteeinrichtung 17. In dem dritten Verfahrensschritt werden nicht sämtliche auf dem ersten Datenspeicher 5 gespeicherte Daten an die Auswerteeinrichtung 17 übermittelt, sondern nur die noch nicht übermittelten Daten.

In einem vierten Verfahrensschritt 38 erfolgt dann eine Auswertung des Datenpakets durch die Auswerteeinrichtung 17, und es wird in Abhängigkeit eines so detektierten Systemzustandes eine Handlungsaufforderung generiert. In einem fünften Verfahrensschritt kann dann die Handlungsaufforderung an die Synchronisationsstation 1 übermittelt werden.

Dieses Verfahren soll nachfolgend am Beispiel des Systemzuverlässigkeitsparameters Luftvolumenstrom erläutert werden. Durch einen Sensor des Inhalators 2 wird bei jedem Inhalationszug der Luftvolumenstrom erfasst und auf dem zweiten Datenspeicher 15 des Inhalators 2 gespeichert. Sobald ein Inhalationsvorgang, der typischerweise bis zu 20 Inhalationszüge umfasst, beendet ist, wird der Inhalator 2 in die Aufnahme 3 der Synchronisationsstation 1 gesetzt, wodurch die gesammelten Parameter betreffend den Luftvolumenstrom über die Inhalatorschnittstelle 8 auf den ersten Datenspeicher 5 übertragen werden; während dieses Speichervorgangs werden die übermittelten Daten mit einem Zeitstempel versehen. Hierzu werden die durch die Hardware-Uhr 31 generierten Daten genutzt. Sobald die Drahtlosschnittstelle 7 mit der Basisstation 11 verbunden ist, werden die Daten an die Auswerteeinrichtung 17 übermittelt. Dort kann eine Analyse des Systemzuverlässigkeitsparameters Luftvolumenstrom vorgenommen werden. Eine Abweichung vom Normalzustand kann beispielsweise dann vorliegen, wenn der Luftvolumenstrom über einen bestimmten Zeitraum hinweg einen bestimmten Schwellwert unterschreitet. Je nach Abweichung kann durch die Auswerteeinrichtung 17 aufgrund historischer Daten auch eine wahrscheinliche Ursache für die Abweichung festgestellt werden. Diese könnte beispielsweise darin bestehen, dass der Inhalator 2 verunreinigt ist. Als Handlungsempfehlung wird die Auswerteeinrichtung 17 dann einen Befehl für das Reinigen des Inhalators 2 über die Basisstation 11 an die Synchronisationsstation 1 übermitteln. Dort kann dem Benutzer beispielsweise über eine Ausgabeeinrichtung 30 in Form eines Displays angezeigt werden, dass eine Reinigung des Inhalators 2 erforderlich ist. Auf diese Weise kann eine gleichbleibend hohe Qualität des Inhalationsvorgangs sichergestellt werden.

## Patentansprüche

1. Tragbare Synchronisationsstation (1) für einen Inhalator (2), vorzugsweise für ein elektronisches Zigarettenprodukt, umfassend folgende Komponenten
- einen ersten Energiespeicher (4),
- einen ersten Datenspeicher (5),
- einen ersten Prozessor (6),
- eine Aufnahme (3) zum Einsetzen des Inhalators (2), und
- eine Inhalatorschnittstelle (8), die dazu eingerichtet ist, eine Datenverbindung mit dem Inhalator (2) herzustellen, wenn der Inhalator (2) in die Aufnahme (3) eingesetzt ist, wobei
- als weitere Komponente eine Drahtlosschnittstelle (7) vorgesehen ist, die dazu eingerichtet ist, Daten an eine entfernte Basisstation (11) zu übermitteln, wobei
- die Synchronisationsstation (1) dazu eingerichtet ist, die Drahtlosschnittstelle (7) derart zu steuern, dass regelmäßig automatische Verbindungsversuche mit der Basisstation (11) unternommen werden, und eine definierte Datenmenge der in dem ersten Datenspeicher (5) gespeicherten Daten an die Basisstation (11) übermittelt wird, sobald eine Verbindung mit der Basisstation (11) besteht, **dadurch gekennzeichnet, dass**
- die Synchronisationsstation (1) dazu eingerichtet ist, die auf dem ersten Datenspeicher (5) gespeicherten Daten mit einem Zeitstempel zu versehen, wobei
- die Daten einen oder mehrere der nachfolgenden Systemzuverlässigkeitsparameter umfassen
- Betriebsdauer des Inhalators (2),
- Benutzungszyklen des Inhalators (2),
- Betriebstemperatur des Inhalators (2),
- Ladezyklen des Inhalators (2),
- Ladezustand des Inhalators (2),
- Betriebsdruck des Inhalators (2), und/oder
- Durchflussmenge des Inhalators (2).

2. Tragbare Synchronisationsstation (1) nach Anspruch 1,
**dadurch gekennzeichnet, dass** die Synchronisationsstation (1) dazu eingerichtet ist, die bereits über die Drahtlosschnittstelle (7) an die Basisstation (11) übermittelten Daten auf dem ersten Datenspeicher (5) kenntlich zu machen, und bei einem erneuten Verbindungsaufbau der Drahtlosschnittstelle (7) mit der Basisstation (11) nur die bislang noch nicht übermittelten Daten an die Basisstation (11) zu übermitteln.

3. Tragbare Synchronisationsstation (1) nach einem der vorangehenden Ansprüche, **dadurch gekennzeichnet, dass** die Synchronisationsstation (1) dazu eingerichtet ist, als unabhängiges Internetendgerät betreiben zu werden.

4. Tragbare Synchronisationsstation (1) nach einem der vorangehenden Ansprüche, **dadurch gekennzeichnet, dass** die Daten einen oder mehrere der nachfolgenden Systemzuverlässigkeitsparameter umfassen
- Betriebsdauer der Synchronisationsstation (1),
- Benutzungszyklen der Synchronisationsstation (1),
- Betriebstemperatur der Synchronisationsstation (1),
- Ladezyklen der Synchronisationsstation (1), und/oder
- Ladezustand der Synchronisationsstation (1).

5. Tragbare Synchronisationsstation (1) nach einem der vorangehenden Ansprüche, **dadurch gekennzeichnet, dass** die Drahtlosschnittstelle (7) eine oder mehrere der folgenden Schnittstellen umfasst:
- eine Mobilfunkschnittstelle,
- eine Bluetooth-Schnittstelle, und/oder
- eine WiFi-Schnittstelle.

6. Tragbare Synchronisationsstation (1) nach einem der vorangehenden Ansprüche, **dadurch gekennzeichnet, dass** Verbindungsinformationen zum Herstellen der Verbindung zwischen der Drahtlosschnittstelle (7) und der Basisstation (11) vorgespeichert sind.

7. Tragbare Synchronisationsstation (1) nach einem der vorangehenden Ansprüche, **dadurch gekennzeichnet, dass**
- eine mechanische Schaltvorrichtung (12) vorgesehen ist, über die die Drahtlosschnittstelle (7) deaktivierbar und/oder deren Verbindung zu den übrigen Komponenten trennbar ist.

8. Tragbare Synchronisationsstation (1) nach einem der vorangehenden Ansprüche, **dadurch gekennzeichnet, dass** die Komponenten innerhalb eines gemeinsamen Gehäuses (10) angeordnet und mit einer gemeinsamen Trägerstruktur (9) fest verbunden sind.

9. Tragbare Synchronisationsstation (1) nach einem der vorangehenden Ansprüche, **dadurch gekennzeichnet, dass** es sich bei der Inhalatorschnittstelle (8) um eine Kontakt-Schnittstelle, vorzugsweise um eine USB-Schnittstelle, handelt.

10. Tragbares Synchronisationssystem (13) umfassend die Synchronisationsstation (1) nach einem der vorangehenden Ansprüche und einen Inhalator (2) umfassend einen zweiten Energiespeicher (14), einen zweiten Prozessor (15) und einen zweiten Datenspeicher (16).

11. Tragbares Synchronisationssystem (13) nach Anspruch 10,
**dadurch gekennzeichnet, dass** das Speichervolumen des ersten Datenspeichers (5) mindestens dem 10-fachen des Speichervolumens des zweiten Datenspeichers (15) entspricht.

12. Tragbares Synchronisationssystem (13) nach einem der Ansprüche 10 oder 11, **dadurch gekennzeichnet, dass** das Synchronisationssystem (13) dazu eingerichtet ist, die auf dem zweiten Datenspeicher (15) gespeicherten Daten über die Inhalatorschnittstelle (8) automatisch auf den ersten Datenspeicher (5) zu übertragen, wenn der Inhalator (2) in die Aufnahme (3) eingesetzt ist.

13. Verfahren zur Detektion des Systemzustands eines Synchronisationssystems (13) nach einem der Ansprüche 10 bis 12, **dadurch gekennzeichnet, dass**
- in einem ersten Verfahrensschritt (35), wenigstens ein Systemzuverlässigkeitsparameter des Inhalators (2) automatisch über die Inhalatorschnittstelle (8) an die Synchronisationsstation (1) übermittelt und auf dem ersten z r Datenspeicher (5) gespeichert wird, wenn der Inhalator (2) in die Aufnahme (3) eingesetzt ist,
- in einem zweiten Verfahrensschritt (36) die Systemzuverlässigkeitsparameter des Inhalators (2) mit einem Zeitstempel versehen werden und diese in einem zu versendenden Datenpaket zusammengefasst werden,
- in einem dritten Verfahrensschritt (37) das Datenpaket über die Drahtlosschnittstelle (7) und die Basisstation (11) an eine Auswerteeinrichtung (17) übermittelt wird,
- in einem vierten Verfahrensschritt (38) eine Auswertung des Datenpakets erfolgt und in Abhängigkeit eines so detektierten Systemzustandes eine Handlungsaufforderung generiert wird.

14. Verfahren nach Anspruch 13, **dadurch gekennzeichnet, dass** in einem fünften Verfahrensschritt (39) die Handlungsaufforderung an die Synchronisationsstation (1) übermittelt wird.

## Claims

1. Portable synchronisation station (1) for an inhaler (2), preferably for an electronic cigarette product, comprising the following components:
- a first energy store (4),
- a first data store (5),
- a first processor (6),
- a receptacle (3) for inserting the inhaler (2), and
- an inhaler interface (8) which is designed to establish a data connection with the inhaler (2) when the inhaler (2) is inserted into the receptacle (3), wherein
- a wireless interface (7) is provided as a further component, which is designed to transmit data to a remote base station (11), wherein
- the synchronisation station (1) is designed to control the wireless interface (7) in such a way that automatic connection attempts with the base station (11) are made regularly, and a defined data volume of the data stored in the first data store (5) is transmitted to the base station (11) as soon as a connection with the base station (11) exists, **characterised in that**
- the synchronisation station (1) is designed to provide the data stored on the first data store (5) with a time stamp, wherein
- the data comprise one or more of the following system reliability parameters:
- operating time of the inhaler (2),
- usage cycles of the inhaler (2),
- operating temperature of the inhaler (2),
- charging cycles of the inhaler (2),
- charging state of the inhaler (2),
- operating pressure of the inhaler (2), and/or
- flow rate of the inhaler (2).

2. Portable synchronisation station (1) according to claim 1,
**characterised in that** the synchronisation station (1) is designed to identify the data already transmitted to the base station (11) via the wireless interface (7) on the first data memory (5), and to transmit only the not yet transmitted data to the base station (11) when the connection of the wireless interface (7) to the base station (11) is re-established.

3. Portable synchronisation station (1) according to any of the preceding claims, **characterised in that** the synchronisation station (1) is designed to be operated as an independent Internet terminal.

4. Portable synchronisation station (1) according to any of the preceding claims, **characterised in that** the data comprise one or more of the following system reliability parameters:
- operating time of the synchronisation station (1),
- usage cycles of the synchronisation station (1),
- operating temperature of the synchronisation station (1),
- charging cycles of the synchronisation station (1), and/or
- charging status of the synchronisation station (1).

5. Portable synchronisation station (1) according to any of the preceding claims, **characterised in that** the wireless interface (7) comprises one or more of the following interfaces:
- a mobile communications interface,
- a Bluetooth interface, and/or
- a Wi-Fi interface.

6. Portable synchronisation station (1) according to any of the preceding claims, **characterised in that** connection information for establishing the connection between the wireless interface (7) and the base station (11) is prestored.

7. Portable synchronisation station (1) according to any of the preceding claims, **characterised in that**
- a mechanical switching apparatus (12) is provided, via which the wireless interface (7) can be deactivated and/or its connection to the other components can be severed.

8. Portable synchronisation station (1) according to any of the preceding claims, **characterised in that** the components are arranged within a common housing (10) and are fixedly connected to a common support structure (9).

9. Portable synchronisation station (1) according to any of the preceding claims, **characterised in that** the inhaler interface (8) is a contact interface, preferably a USB interface.

10. Portable synchronisation system (13) comprising the synchronisation station (1) according to any of the preceding claims and an inhaler (2) comprising a second energy store (14), a second processor (15) and a second data store (16).

11. Portable synchronisation system (13) according to claim 10,
**characterised in that** the storage volume of the first data store (5) corresponds to at least 10 times the storage volume of the second data store (15).

12. Portable synchronisation system (13) according to any of claims 10 or 11,
**characterised in that** the synchronisation system (13) is designed to automatically transfer the data stored on the second data store (15) to the first data store (5) via the inhaler interface (8) when the inhaler (2) is inserted into the receptacle (3).

13. Method for detecting the system state of a synchronisation system (13) according to any of claims 10 to 12,
**characterised in that**
- in a first method step (35), at least one system reliability parameter of the inhaler (2) is automatically transmitted via the inhaler interface (8) to the synchronisation station (1) and stored on the first data store (5) when the inhaler (2) is inserted into the receptacle (3),
- in a second method step (36), the system reliability parameters of the inhaler (2) are provided with a time stamp and these are combined in a data packet to be sent,
- in a third method step (37), the data packet is transmitted via the wireless interface (7) and the base station (11) to an evaluation device (17),
- in a fourth method step (38), the data packet is evaluated and an action request is generated depending on a system state detected in this way.

14. Method according to claim 13, **characterised in that,** in a fifth method step (39), the action request is transmitted to the synchronisation station (1).

## Revendications

1. Station de synchronisation portable (1) pour un inhalateur (2), de préférence pour un produit de type cigarette électronique, comprenant les composants suivants :
- un premier accumulateur d'énergie (4),
- une première mémoire de données (5),
- un premier processeur (6),
- un logement (3) pour insérer l'inhalateur (2), et
- une interface d'inhalateur (8) qui est conçue pour établir une liaison de données avec l'inhalateur (2) lorsque l'inhalateur (2) est inséré dans le logement (3), dans laquelle
- il est prévu comme autre composant une interface sans fil (7) qui est conçue pour transmettre des données à une station de base éloignée (11), dans laquelle
- la station de synchronisation (1) est conçue pour commander l'interface sans fil (7) de telle sorte que des tentatives de liaison automatiques avec la station de base (11) soient régulièrement entreprises, et qu'une quantité définie de données stockées dans la première mémoire de données (5) soit transmise à la station de base (11) dès qu'une liaison est établie avec la station de base (11), **caractérisée en ce que**
- la station de synchronisation (1) est conçue pour munir les données stockées dans la première mémoire de données (5) d'un horodatage, dans laquelle
- les données comprennent un ou plusieurs des paramètres de fiabilité du système suivants :
- durée de fonctionnement de l'inhalateur (2),
- cycles d'utilisation de l'inhalateur (2),
- température de fonctionnement de l'inhalateur (2),
- cycles de charge de l'inhalateur (2),
- état de charge de l'inhalateur (2),
- pression de service de l'inhalateur (2), et/ou
- débit de l'inhalateur (2).

2. Station de synchronisation portable (1) selon la revendication 1, **caractérisée en ce que** la station de synchronisation (1) est conçue pour rendre reconnaissables dans la première mémoire de données (5) les données déjà transmises à la station de base (11) via l'interface sans fil (7), et pour ne transmettre à la station de base (11), lors d'un nouvel établissement de liaison de l'interface sans fil (7) avec la station de base (11), que les données qui n'ont pas encore été transmises jusqu'à présent.

3. Station de synchronisation portable (1) selon l'une des revendications précédentes, **caractérisée en ce que** la station de synchronisation (1) est conçue pour fonctionner comme un terminal Internet indépendant.

4. Station de synchronisation portable (1) selon l'une des revendications précédentes, **caractérisée en ce que** les données comprennent un ou plusieurs des paramètres de fiabilité du système suivants :
- durée de fonctionnement de la station de synchronisation (1),
- cycles d'utilisation de la station de synchronisation (1),
- température de fonctionnement de la station de synchronisation (1),
- cycles de charge de la station de synchronisation (1), et/ou
- état de charge de la station de synchronisation (1).

5. Station de synchronisation portable (1) selon l'une des revendications précédentes, **caractérisée en ce que** l'interface sans fil (7) comprend une ou plusieurs des interfaces suivantes :
- une interface de téléphonie mobile,
- une interface Bluetooth, et/ou
- une interface Wi-Fi.

6. Station de synchronisation portable (1) selon l'une des revendications précédentes, **caractérisée en ce que** des informations de liaison sont préenregistrées pour établir la liaison entre l'interface sans fil (7) et la station de base (11).

7. Station de synchronisation portable (1) selon l'une des revendications précédentes, **caractérisée en ce que**
- il est prévu un dispositif de commutation mécanique (12) qui permet de désactiver l'interface sans fil (7) et/ou de couper sa liaison avec les autres composants.

8. Station de synchronisation portable (1) selon l'une des revendications précédentes, **caractérisée en ce que** les composants sont disposés à l'intérieur d'un boîtier commun (10) et reliés de manière fixe à une structure de support commune (9).

9. Station de synchronisation portable (1) selon l'une des revendications précédentes, **caractérisée en ce que** l'interface d'inhalateur (8) est une interface de contact, de préférence une interface USB.

10. Système de synchronisation portable (13) comprenant la station de synchronisation (1) selon l'une des revendications précédentes et un inhalateur (2) comprenant un deuxième accumulateur d'énergie (14), un deuxième processeur (15) et une deuxième mémoire de données (16).

11. Système de synchronisation portable (13) selon la revendication 10, **caractérisé en ce que** le volume de stockage de la première mémoire de données (5) correspond au moins à 10 fois le volume de stockage de la deuxième mémoire de données (15).

12. Système de synchronisation portable (13) selon l'une des revendications 10 ou 11, **caractérisé en ce que** le système de synchronisation (13) est conçu pour transférer automatiquement les données stockées dans la deuxième mémoire de données (15) vers la première mémoire de données (5) via l'interface d'inhalateur (8) lorsque l'inhalateur (2) est inséré dans le logement (3).

13. Procédé de détection de l'état d'un système de synchronisation (13) selon l'une des revendications 10 à 12, **caractérisé en ce que**
- dans une première étape de procédé (35), au moins un paramètre de fiabilité du système de l'inhalateur (2) est transmis automatiquement à la station de synchronisation (1) via l'interface d'inhalateur (8) et stocké dans la première mémoire de données (5) lorsque l'inhalateur (2) est inséré dans le logement (3),
- dans une deuxième étape de procédé (36), les paramètres de fiabilité du système de l'inhalateur (2) sont munis d'un horodatage et regroupés dans un paquet de données à envoyer,
- dans une troisième étape de procédé (37), le paquet de données est transmis à un dispositif d'évaluation (17) via l'interface sans fil (7) et la station de base (11),
- dans une quatrième étape de procédé (38), une évaluation du paquet de données est effectuée et une demande d'action est générée en fonction d'un état du système ainsi détecté.

14. Procédé selon la revendication 13, **caractérisé en ce que** dans une cinquième étape de procédé (39), la demande d'action est transmise à la station de synchronisation (1).
